# EUROPEAN PATENT APPLICATION

(11) **EP 4 042 959 A1**
(43) Date of publication of application: **17.08.2022**
(21) Application number: 20870622.6
(22) Date of filing: 29.09.2020
(51) Int. Cl.: A61B 18/12, A61B 18/14

(54) **ENERGY GENERATING DEVICE AND CAUTERIZATION SYSTEM**

(30) Priority: 30.09.2019 JP 2019179469
(71) Applicant: TERUMO Kabushiki Kaisha, Tokyo 151-0072 (JP)
(72) Inventor: TAKAHASHI, Yusuke, Ashigarakami-gun, Kanagawa 259-0151 (JP); KATOU, Yukitoshi, Ashigarakami-gun, Kanagawa 259-0151 (JP); TAKEMURA, Tomoaki, Ashigarakami-gun, Kanagawa 259-0151 (JP)
(74) Representative: Casalonga
(86) International application number: PCT/JP2020/036818
(87) International publication number: WO 2021/065876

(57) **Abstract**

Provided are an energy generating device and a cauterization system which make it possible to uniformly cauterize bio tissues at a desired damage level and to reduce tissue damage or generation of distal embolization due to clot formation. This energy generating device (100) comprises a control unit (130) for controlling electric energy to be outputted to electrodes (22A-22D) for use in cauterizing bio tissues, wherein the control unit (130) has: an impedance calculation unit (132) which calculates impedance between an electrode and an counter electrode opposite thereto; and an output adjustment unit (131) which decreases electric power to the electrodes in the case when the impedance calculated by the impedance calculation unit (132) becomes equal to or greater than a predetermined threshold value.

## Description

### Technical Field

The present invention relates to an energy generating device that supplies energy to an electrode for cauterization, and to a cauterization system.

### Background Art

In recent years, a cauterization device has been used which includes an electrode that cauterizes a biological tissue in a living body. Examples of such a cauterization device include a device that cauterizes the vicinity of a through-hole formed in an atrial septum, to maintain a penetrated state, and a device that cauterizes a myocardium to treat arrhythmia.

When a biological tissue is cauterized by the aforementioned cauterization device, it is difficult to detect how much energy is applied to the biological tissue. For this reason, energy cannot be applied to the biological tissue with high reproducibility, and the treatment effect varies.

For this reason, PTL 1 discloses a cauterization state notification device that acquires an impedance of a myocardium from an electrode which performs cauterization, and changes notification sound based on a change in the acquired impedance, in order to treat arrhythmia. An operator can easily determine the state of cauterization from the notification sound.

### Citation List

### Patent Literature

PTL 1: JP-A-2013-111332

### Summary of Invention

### Technical Problem

The cauterization state notification device disclosed in PTL 1 makes notification about the state of cauterization by notification sound, and the operator needs to make a determination from the notification sound and to continue or stop the cauterization. For this reason, damage levels of a biological tissue may be not uniform, or tissue damage caused by the application of excessive energy or peripheral embolism caused by the formation of blood clots may be generated.

The invention is conceived in view of the aforementioned problems, and an object of the invention is to provide an energy generating device and a cauterization system capable of uniformly cauterizing a biological tissue at a desired damage level, and reducing the generation of tissue damage or the generation of peripheral embolism caused by the formation of blood clots.

### Solution to Problem

According to one aspect of the invention to achieve the object, there is provided a cauterization device which is an energy generating device including: a control unit that is configured to control an electric energy output to at least one electrode that is configured to cauterize a biological tissue. The control unit includes an impedance calculation unit that is configured to calculate an impedance between the electrode and an electrode serving as a return electrode paired with the electrode, and an output adjustment unit that is configured cause electric power to the electrodes to decrease when the impedance calculated by the impedance calculation unit is greater a predetermined threshold value or is equal to or greater is the threshold value or more.

According to another aspect of the invention to achieve the object, there is provided a cauterization system including: the energy generating device; and a cauterization device including at least one electrode that is configured to cauterize a biological tissue.

### Advantageous Effect of Invention

The energy generating device and the cauterization system configured as described above can determine a level of damage to the tissue caused by the electrode, based on an increase in the impedance of the biological tissue caused by the cauterization, and cause the electric power to the electrode to automatically decrease. For this reason, the energy generating device can uniformly cauterize the biological tissue at a desired damage level. In addition, it is possible to reduce the generation of tissue damage caused by the application of excessive energy, or the generation of peripheral embolism caused by the formation of blood clots.

The output adjustment unit may cause the electric power between the electrode and the electrode serving as a return electrode of the electrode, to be maintained substantially constant when the impedance calculated by the impedance calculation unit is the threshold value or less or is less than the threshold value. Accordingly, before the impedance reaches the threshold value, the energy generating device can automatically decrease current according to the impedance that gradually increases as the cauterization progresses. For this reason, the energy generating device can facilitate uniform cauterization of the biological tissue at a desired damage level. In addition, it is possible to reduce the generation of tissue damage caused by the application of excessive energy, or the generation of peripheral embolism caused by the formation of blood clots.

The output adjustment unit may cause a voltage between the electrode and the electrode serving as a return electrode of the electrode, to be maintained substantially constant when the impedance calculated by the impedance calculation unit is the threshold value or less or is less than the threshold value. Accordingly, before the impedance reaches the threshold value, the energy generating device can automatically decrease electric power according to the impedance that gradually increases as the cauterization progresses. For this reason, the energy generating device can facilitate uniform cauterization of the biological tissue at a desired damage level. In addition, it is possible to reduce the generation of tissue damage caused by the application of excessive energy, or the generation of peripheral embolism caused by the formation of blood clots.

The cauterization device may include a pressing mechanism on which the electrode is disposed and by which the electrode is pressable against the biological tissue. Accordingly, the electrode comes into close contact with the biological tissue, and is unlikely to be exposed in blood. For this reason, it is possible to suppress the generation of blood clots or the like caused by the leakage of a current to the blood. In addition, since the electrode comes into close contact with the biological tissue, a change in impedance can be accurately detected, so that a damage level of the tissue can be accurately determined. For this reason, the cauterization system can uniformly cauterize the biological tissue at a desired damage level.

The cauterization device may include a shaft portion that is elongate. The pressing mechanism may include an expansion body provided at a distal portion of the shaft portion to be expandable and contractable in a radial direction. The expansion body may include a plurality of wire portions connected to the shaft portion, and at least one holding portion formed of at least one wire portion. The holding portion may include a distal side holding portion and a proximal side holding portion between which a separated distance is narrowed when the expansion body expands. The electrode may be disposed on the distal side holding portion and/or on the proximal side holding portion. Accordingly, the cauterization system effectively brings the electrode into close contact with the biological tissue using the expansion body, so that the electrode can be unlikely to be exposed in the blood. For this reason, it is possible to reduce the generation of peripheral embolism caused by the formation of blood clots.

The cauterization device may include a shaft portion that is elongate. The pressing mechanism may include a balloon provided at a distal portion of the shaft portion to be inflatable and contractable in a radial direction. The electrode may be disposed on an outer surface of the balloon. Accordingly, the cauterization system effectively brings the electrode into close contact with the biological tissue using the balloon, so that the electrode can be unlikely to be exposed in the blood. For this reason, it is possible to reduce the generation of peripheral embolism caused by the formation of blood clots.

The cauterization device may be a device that cauterizes a biological tissue in the vicinity of a through-hole to maintain the through-hole formed in an atrial septum. Accordingly, the cauterization system can uniformly cauterize the biological tissue in the vicinity of the through-hole of the atrial septum at a desired damage level to properly maintain the through-hole.

### Brief Description of Drawings

[Fig. 1] Fig. 1 is a front view illustrating an overall configuration of a cauterization system according to a first embodiment.
[Fig. 2] Fig. 2 is an enlarged perspective view of a distal portion of a cauterization device.
[Fig. 3] Fig. 3 is a block diagram of the cauterization system according to the first embodiment.
[Fig. 4] Fig. 4 is a view for describing a treatment method using the cauterization system according to the first embodiment, and is a view for schematically describing a state where an expansion body is disposed in a through-hole of an atrial septum, in which the cauterization device and a biological tissue are illustrated in a front view and in a cross-sectional view, respectively.
[Fig. 5] Fig. 5 is a view for schematically describing a state where the expansion body is disposed in the atrial septum, in which the cauterization device and the biological tissue are illustrated in a front view and in a cross-sectional view, respectively.
[Fig. 6] Fig. 6 is a view for schematically describing a state where the expansion body is expanded in diameter in the atrial septum, in which the cauterization device and the biological tissue are illustrated in a front view and in a cross-sectional view, respectively.
[Fig. 7] Fig. 7 is a flowchart illustrating a flow of control in a control unit.
[Fig. 8] Fig. 8 is a graph to be displayed on a notification unit.
[Fig. 9] Fig. 9 illustrates front views of the vicinities of holding portions of cauterization systems according to modification examples, Fig. 9(A) illustrates a first modification example, and Fig. 9(B) illustrates a second modification example.
[Fig. 10] Fig. 10 is a block diagram illustrating a third modification example of the cauterization system according to the first embodiment.
[Fig. 11] Fig. 11 is a front view illustrating an overall configuration of a cauterization system according to a second embodiment.
[Fig. 12] Fig. 12 is a block diagram of the cauterization system according to the second embodiment.
[Fig. 13] Fig. 13 is a view for describing a treatment method using the cauterization system according to the second embodiment, and is a view for schematically describing a state where an expansion body is disposed in a through-hole of an atrial septum, in which a cauterization device and a biological tissue are illustrated in a front view and in a cross-sectional view, respectively.
[Fig. 14] Fig. 14 illustrates views for describing a treatment method using the cauterization system according to the second embodiment, Fig. 14(A) illustrates a state where the balloon is inflated, and Fig. 14(B) is a cross-sectional view taken along line A-A in Fig. 14(A).
[Fig. 15] Fig. 15 is a front view of the vicinity of an expansion body of a modification example.

### Description of Embodiments

Hereinafter, embodiments of the invention will be described with reference to the accompanying drawings. Note that dimensional ratios in the drawings may be exaggerated and different from actual ratios for convenience of description. In addition, in the specification, a side on which a cauterization device 10 is inserted into a biological lumen will be referred to as a "distal side", and a side on which operation is performed will be referred to as a "proximal side".

### <First Embodiment>

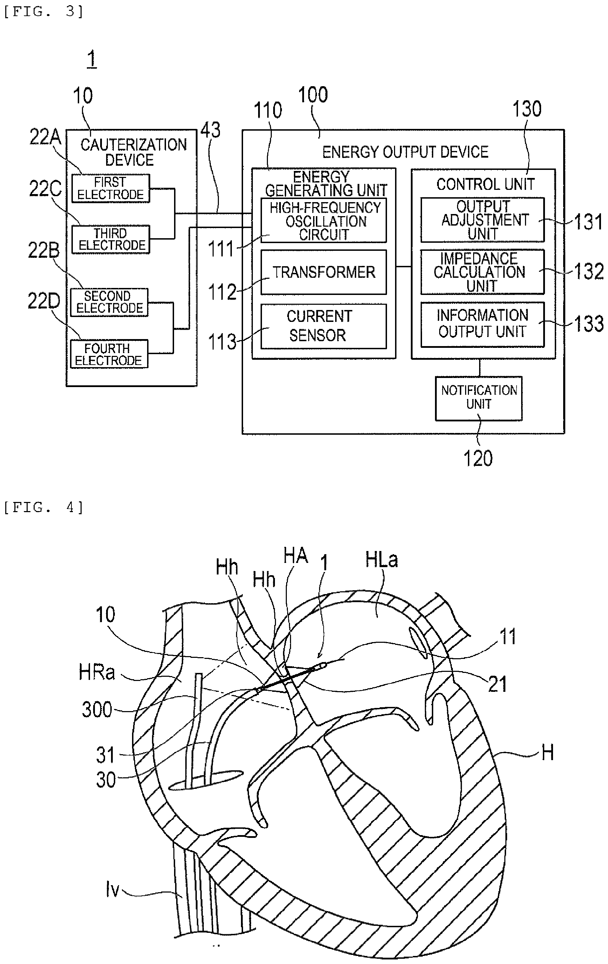

As illustrated in Fig. 4, a cauterization system 1 according to a first embodiment is configured to be able to expand a through-hole Hh formed in an atrial septum HA of a heart H of a patient and to perform cauterization to maintain the size of the expanded through-hole Hh. The cauterization system 1 includes the cauterization device 10 that is inserted into a living body to cauterize a biological tissue, and an energy generating device 100 that supplies electric energy to the cauterization device 10.

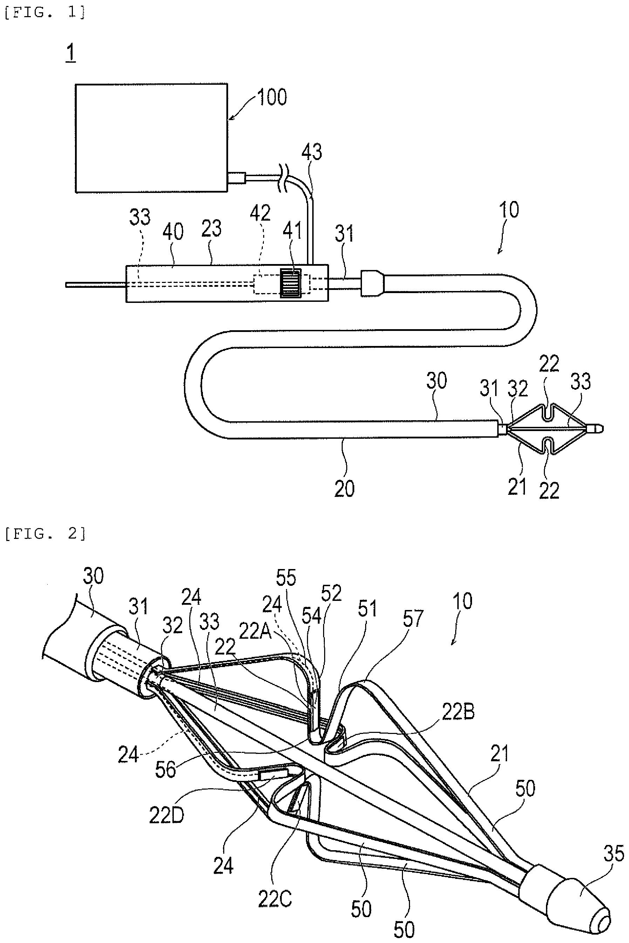

Initially, the cauterization device 10 will be described. As illustrated in Figs. 1 and 2, the cauterization device 10 includes a shaft portion 20 that is elongate, an expansion body 21 provided at a distal portion of the shaft portion 20, and an operation unit 23 provided at a proximal portion of the shaft portion 20. The expansion body 21 is provided with an energy output unit 22 for performing cauterization.

The shaft portion 20 includes an outer shaft 31 that holds the expansion body 21 at the distal portion of the outer shaft 31, and a storage sheath 30 that stores the outer shaft 31. The storage sheath 30 is movable forward and backward with respect to the outer shaft 31 in an axial direction. In a state where the storage sheath 30 is moved to a distal side of the shaft portion 20, the storage sheath 30 can store the expansion body 21 thereinside. The storage sheath 30 is moved to the proximal side from a state where the expansion body 21 is stored, and thus the expansion body 21 can be exposed.

A pulling shaft 33 is stored inside the outer shaft 31. The pulling shaft 33 is a shaft for pulling to act a compression force on the expansion body 21. The pulling shaft 33 protrudes from a distal end of the outer shaft 31 to the distal side, and a distal portion of the pulling shaft 33 is fixed to a distal member 35. A proximal portion of the pulling shaft 33 is led out to the proximal side from the operation unit 23. The distal member 35 to which the distal portion of the pulling shaft 33 is fixed may not be fixed to the expansion body 21. Accordingly, the distal member 35 can pull the expansion body 21 in a compression direction. In addition, when the expansion body 21 is stored in the storage sheath 30, the distal member 35 is separated to the distal side from the expansion body 21, so that the expansion body 21 can be easily moved in a stretching direction and storability can be improved.

The operation unit 23 includes a housing 40 to be gripped by an operator, an operation dial 41 to be rotationally operable by the operator, a conversion mechanism 42 that operates in conjunction with rotation of the operation dial 41, and a connector 43 that is connectable to the energy generating device 100. The pulling shaft 33 is held by the conversion mechanism 42 inside the operation unit 23. The conversion mechanism 42 can move the held pulling shaft 33 forward and backward along the axial direction with rotation of the operation dial 41. For example, a rack and pinion mechanism can be used as the conversion mechanism 42. The connector 43 can be connected to the energy generating device 100, and receive electric energy from the energy generating device 100. It is preferable that the connector 43 is attachable to and detachable from the energy generating device 100.

The expansion body 21 includes a plurality of wire portions 50 in a circumferential direction. In the present embodiment, four wire portions 50 are provided in the circumferential direction. Note that the number of the wire portions 50 is not particularly limited. Each of the wire portions 50 is expandable and contractable in a radial direction of the expansion body 21. In a natural state where no external force acts on the expansion body 21, the expansion body 21 is in a reference form where the expansion body 21 is deployed in the radial direction. A proximal portion of the wire portion 50 extends from a distal portion of the outer shaft 31 to the distal side. A distal portion of the wire portion 50 extends from a proximal portion of the distal member 35 to the proximal side. The wire portion 50 is inclined such that the size in the radial direction increases from both end portions toward a central portion in the axial direction. In addition, the wire portion 50 includes a holding portion 51 having a valley shape in the radial direction of the expansion body 21, at the central portion of the wire portion 50 in the axial direction.

The holding portion 51 includes a proximal side holding portion 52, and a distal side holding portion 53 located closer to the distal side than the proximal side holding portion 52. The holding portion 51 further includes a proximal side outward projection portion 55, an inward projection portion 56, and a distal side outward projection portion 57. It is preferable that in the reference form, an interval between the proximal side holding portion 52 and the distal side holding portion 53 in the axial direction is slightly wider on a radially outward side than on a radially inward side. Accordingly, a biological tissue is easily disposed between the proximal side holding portion 52 and the distal side holding portion 53 from the radially outward side.

The proximal side holding portion 52 includes a projection portion 54 protruding toward the distal side. The energy output unit 22 is disposed on the projection portion 54. Note that the proximal side holding portion 52 may not include the projection portion 54. Namely, the energy output unit 22 may not protrude to the distal side.

The proximal side outward projection portion 55 is located on a proximal side of the proximal side holding portion 52, and is formed in a shape projecting outward in the radial direction. The distal side outward projection portion 57 is located on a distal side of the distal side holding portion 53, and is formed in a shape projecting outward in the radial direction. The inward projection portion 56 is located between the proximal side holding portion 52 and the distal side holding portion 53, and is formed in a shape projecting inward in the radial direction. The proximal side outward projection portion 55, the inward projection portion 56, and the distal side outward projection portion 57 are stored in the storage sheath 30, thus being deformable from a projection shape into a shape close to being flat.

In the present embodiment, the energy output unit 22 is provided at the proximal side holding portion 52, but the energy output unit 22 may be provided at the distal side holding portion 53. In addition, the energy output units 22 may be provided at both the proximal side holding portion 52 and the distal side holding portion 53.

Each of the wire portions 50 forming the expansion body 21 has, for example, a flat plate shape obtained by cutting a cylinder. A wire forming the expansion body 21 can have a thickness of 50 to 500 µm and a width of 0.3 to 2.0 mm. However, a wire forming the expansion body 21 may have dimensions outside these ranges. In addition, the shape of the wire portion 50 is not limited, and may have, for example, a circular cross-sectional shape or other cross-sectional shapes.

As illustrated in Figs. 2 and 3, the energy output unit 22 includes a first electrode 22A, a second electrode 22B, a third electrode 22C, and a fourth electrode 22D. The first electrode 22A, the second electrode 22B, the third electrode 22C, and the fourth electrode 22D are disposed in order on the wire portions 50 arranged in the circumferential direction of the expansion body 21.

Since the energy output unit 22 is provided at the projection portion 54 of the proximal side holding portion 52, when the holding portion 51 holdes the atrial septum HA, energy from the energy output unit 22 is transmitted from a right atrium side to the atrial septum HA. Note that when the energy output unit 22 is provided at the distal side holding portion 53, energy from the energy output unit 22 is transmitted from a left atrium side to the atrial septum HA.

The energy output unit 22 is configured as a bipolar electrode that receives electric energy from the energy generating device 100. The same voltage is applied to the first electrode 22A and to the third electrode 22C. The same voltage is applied to the second electrode 22B and to the fourth electrode 22D. A voltage having a polarity opposite that of the first electrode 22A and of the third electrode 22C is applied to the second electrode 22B and to the fourth electrode 22D. Therefore, energization is performed between the first electrode 22A and the second electrode 22B, between the second electrode 22B and the third electrode 22C, between the third electrode 22C and the fourth electrode 22D, and between the fourth electrode 22D and the first electrode 22A. Electric energy from the connector 43 connected to the energy generating device 100 is supplied to the energy output unit 22 by a conducting wire 24 coated with an insulating coating material. When the energy output unit 22 is a bipolar electrode, the number of the electrodes is not particularly limited as long as the number of the electrodes is 2 or more.

The wire portion 50 can be made of a metallic material. As the metallic material, for example, a titanium-based (Ti-Ni, Ti-Pd, Ti-Nb-Sn, or the like) alloy, a copper-based alloy, stainless steel, β-titanium steel, or a Co-Cr alloy can be used. Note that it is better to use an alloy or the like having a spring property such as a nickel-titanium alloy. However, the material for the wire portion 50 is not limited to these materials, and the wire portion 50 may be made of other materials.

The shaft portion 20 includes an inner shaft 32 inside the outer shaft 31, and the pulling shaft 33 is stored inside the inner shaft 32. A guide wire lumen is formed in the pulling shaft 33 and in the distal member 35 along the axial direction, and a guide wire 11 can be inserted into the guide wire lumen.

It is preferable that the storage sheath 30, the outer shaft 31, and the inner shaft 32 of the shaft portion 20 are made of a material having a certain degree of flexibility. Examples of such a material include polyolefins such as polyethylene, polypropylene, polybutene, ethylene-propylene copolymer, ethylene-vinyl acetate copolymer, ionomer, and a mixture of two or more thereof, fluororesins such as soft polyvinyl chloride resin, polyamide, polyamide elastomer, polyether blockamide, polyester, polyester elastomer, polyurethane, and polytetrafluoroethylene, polyimide, PEEK, silicone rubber, and latex rubber.

The pulling shaft 33 can be made of, for example, an elongate wire such as a metallic material such as stainless steel or a super-elastic alloy such as a nickel-titanium alloy or a copper-zinc alloy, or a resin material having relatively high rigidity. In addition, the pulling shaft 33 may be formed by coating the above material with a resin material such as polyvinyl chloride, polyethylene, polypropylene, ethylene-propylene copolymer, or fluororesin.

The distal member 35 can be made of, for example, a metallic material such as stainless steel or a super-elastic alloy such as a nickel-titanium alloy or a copper-zinc alloy, or a resin material having relatively high rigidity.

Next, the energy generating device 100 will be described. As illustrated in Fig. 3, the energy generating device 100 includes an energy generating unit 110, a notification unit 120, and a control unit 130.

The energy generating unit 110 includes a high-frequency oscillation circuit 111, a transformer 112, and a current sensor 113. The energy generating unit 110 can output electric power input from a commercial power supply, which is converted into a high-frequency current by the high-frequency oscillation circuit 111 and then is converted into an arbitrary voltage by the transformer 112. In addition, the energy generating unit 110 detects values of currents flowing through the electrodes 22A to 22D, using the current sensor 113.

The notification unit 120 is a portion that notifies the operator of a control status. The notification unit 120 is, for example, a speaker that makes notification by sound or an image monitor that makes notification by an image. The image monitor can display graphs of changes in electric power, impedance, voltage, and current over time, together with a threshold value, or can numerically display electric power, impedance, voltage, current, threshold value, elapsed time, and various other parameters. In addition, the image monitor can display control status, warning, and the like. Note that a display method of the image monitor or an output method of the speaker is not particularly limited. Note that the notification unit 120 may be an external device that is communicatably connected to the energy generating device 100, instead of being configured in the energy generating device 100.

The control unit 130 includes a central processing unit (CPU), a storage circuit, and an operation program. The control unit 130 may be connected to an interface such as a keyboard or a mouse. The control unit 130 is, for example, a computer. The control unit 130 includes an output adjustment unit 131, an impedance calculation unit 132, and an information output unit 133.

The output adjustment unit 131 controls the energy generating unit 110 to adjust an output of a high-frequency current from the energy generating unit 110. The output adjustment unit 131 can cause the energy generating unit 110 to output a high-frequency current at an arbitrary voltage.

The impedance calculation unit 132 acquires an output high-frequency voltage value from the energy generating unit 110 or from the output adjustment unit 131, and acquires a high-frequency current value detected by the current sensor 113, from the energy generating unit 110. The impedance calculation unit 132 divides the high-frequency voltage value by the high-frequency current value, to calculate a biological impedance value of a site to be cauterized.

The information output unit 133 transmits information selected from various information such as output electric power, voltage, current, calculated impedance value, threshold value, elapsed time, and operating state of the output adjustment unit 131, to the notification unit 120.

Next, a treatment method using the cauterization system 1 according to the present embodiment will be described. The treatment method is performed on a patient suffering from a heart failure (left heart failure). More specifically, as illustrated in Fig. 4, the treatment method is performed on a patient suffering from a chronic heart failure in which a myocardium of a left ventricle of the heart H is hypertrophied and increases in stiffness (hardness) to cause an increase in blood pressure in a left atrium HLa.

When the operator forms the through-hole Hh, the operator delivers an introducer in which a guiding sheath and a dilator are combined together, to the vicinity of the atrial septum HA. The introducer can be delivered to, for example, a right atrium HRa via an inferior vena cava Iv. In addition, the introducer can be delivered using the guide wire 11. The operator can insert the guide wire 11 into the dilator, and deliver the introducer along the guide wire 11. Note that the insertion of the introducer into or the insertion of the guide wire 11 into a living body can be performed using a known method such as using an introducer for blood vessel introduction.

Next, the operator causes a puncture device (not illustrated) and the dilator to penetrate through the atrial septum HA from a right atrium HRa side toward a left atrium HLa side to form the through-hole Hh. For example, a device such as a wire having a sharp distal end can be used as the puncture device. The puncture device is inserted into a dilator 312, and is delivered to the atrial septum HA. After the guide wire 11 is removed from the dilator, instead of the guide wire 11, the puncture device can be delivered to the atrial septum HA.

Next, the cauterization device 10 is delivered to the vicinity of the atrial septum HA along the guide wire 11 inserted into the left atrium HLa from the right atrium HRa via the through-hole Hh in advance. At this time, a part of a distal portion of the cauterization device 10 passes through the through-hole Hh opened in the atrial septum HA, and reaches the left atrium HLa. When the cauterization device 10 is inserted, the expansion body 21 is in a contracted form where the expansion body 21 is stored in the storage sheath 30. In the contracted form, the proximal side outward projection portion 55, the inward projection portion 56, and the distal side outward projection portion 57 that have a projection shape in a natural state are deformed into a shape close to being flat, so that the expansion body 21 is contracted in the radial direction.

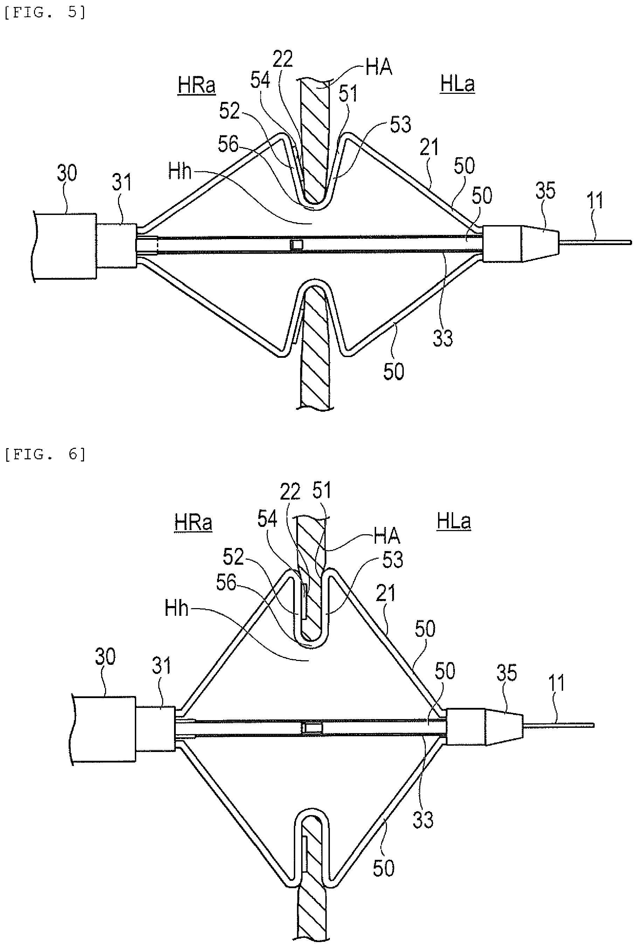

Next, the storage sheath 30 is moved to the proximal side to expose a distal side portion of the expansion body 21 into the left atrium HLa. Accordingly, the distal side portion of the expansion body 21 is deployed in the radial direction inside the left atrium HLa by its own restoring force. Next, as illustrated in Fig. 5, the storage sheath 30 is moved to the proximal side to expose the entirety of the expansion body 21. Accordingly, a proximal side portion of the expansion body 21 is deployed in the radial direction inside the right atrium HRa by its own restoring force. At this time, the inward projection portion 56 is disposed inside the through-hole Hh. Accordingly, the entirety of the expansion body 21 is deployed by its own restoring force, and restores to the original reference form or to a form close to the reference form. At this time, the atrial septum HA is disposed between the proximal side holding portion 52 and the distal side holding portion 53. Note that the expansion body 21 may come into contact with the through-hole Hh, thereby returning to a shape close to the reference form instead of completely returning to the reference form. Note that in this state, the expansion body 21 is not covered with the storage sheath 30 and does not receive a force from the pulling shaft 33. This form of the expansion body 21 can be defined as being included in the reference form.

Next, the operator operates the operation unit 23 in a state where the atrial septum HA is held by the holding portion 51, to move the pulling shaft 33 to the proximal side. Accordingly, as illustrated in Fig. 6, the expansion body 21 receiving a compression force in the axial direction has an expanded form where the expansion body 21 is more expanded in the radial direction than in the reference form. In the expanded form of the expansion body 21, the proximal side holding portion 52 and the distal side holding portion 53 approach each other and the atrial septum HA is holded between the proximal side holding portion 52 and the distal side holding portion 53. The holding portion 51 additionally expands in a state where the holding portion 51 holdes the atrial septum HA, to widen the holded through-hole Hh in the radial direction.

After the through-hole Hh is expanded, hemodynamics is confirmed.
As illustrated in Fig. 4, the operator delivers a hemodynamics confirmation device 300 to the right atrium HRa via the inferior vena cava Iv. For example, a known echo catheter can be used as the hemodynamics confirmation device 300. The operator can cause a display device such as a display to display an echo image acquired by the hemodynamics confirmation device 300, and confirm the amount of blood passing through the through-hole Hh, based on a display result.

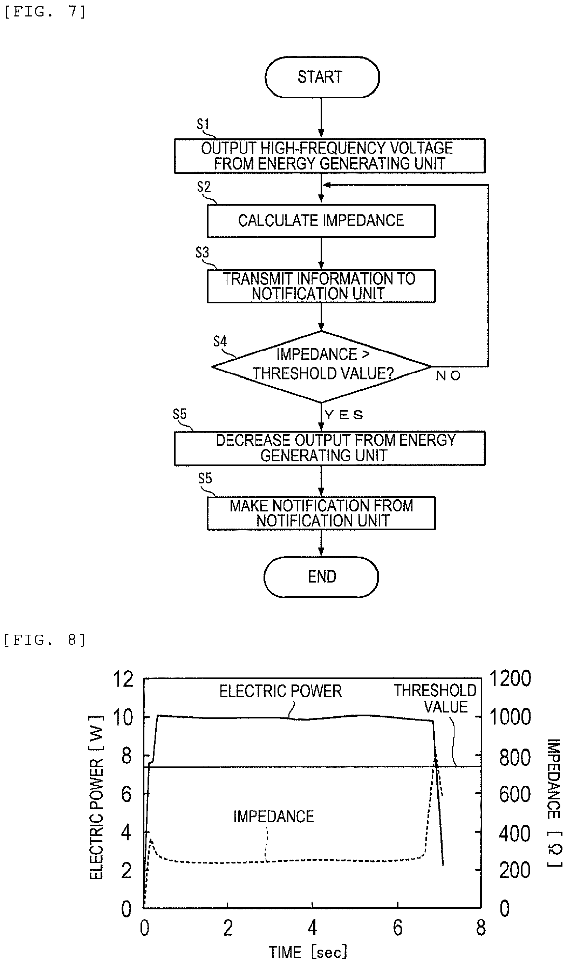

Next, the operator performs a maintenance treatment to maintain the size of the through-hole Hh. In the maintenance treatment, energy is applied to an edge portion of the through-hole Hh through the energy output unit 22, so that the edge portion of the through-hole Hh is cauterized (heated and cauterized) by the energy. Hereinafter, a method of the maintenance treatment using the cauterization system 1 will be described with reference to the flowchart illustrated in Fig. 7.

As illustrated in Figs. 1 and 3, the operator operates the energy generating device 100 to which the connector 43 of the cauterization device 10 is connected, to start cauterization. Accordingly, the output adjustment unit 131 of the control unit 130 causes the energy generating unit 110 to output a high-frequency current at constant electric power (step S1). Note that electric power may not be strictly constant and may vary slightly. In addition, the output adjustment unit 131 may not cause the energy generating unit 110 to output a constant electric power. The first electrode 22A and the third electrode 22C serve as return electrodes of the second electrode 22B and of the fourth electrode 22D. For this reason, energization is performed between the first electrode 22A and the second electrode 22B, between the second electrode 22B and the third electrode 22C, between the third electrode 22C and the fourth electrode 22D, and between the fourth electrode 22D and the first electrode 22A at constant electric power.

The electrodes 22A to 22D are disposed on the projection portions 54 of the proximal side holding portions 52. For this reason, the maintenance treatment is performed in a state where the electrodes 22A to 22D are buried in a biological tissue by pressing the projection portions 54 against the atrial septum HA. Accordingly, the electrodes 22A to 22D do not come into contact with the blood during the maintenance treatment, so that it is possible to suppress the generation of blood clots or the like caused by the leakage of a current to the blood.

When a biological tissue in the vicinity of the edge portion of the through-hole Hh is cauterized through the electrodes 22A to 22D, a degenerated portion in which the biological tissue is degenerated is formed in the vicinity of the edge portion. Since the biological tissue in the degenerated portion is in a state where elasticity is lost, the through-hole Hh can maintain a shape when the through-hole Hh is widened by the expansion body 21. In the cauterized biological tissue, a damage level (degree of cauterization) progresses and the impedance increases.

The impedance calculation unit 132 of the control unit 130 acquires information from the energy generating unit 110 and from the output adjustment unit 131 while electric power is output from the energy generating unit 110, and calculates an impedance (step S2). The information output unit 133 of the control unit 130 transmits information selected from output electric power, voltage, measured current, calculated impedance value, threshold value, elapsed time, and operating state of the output adjustment unit 131, and the like, to the notification unit 120 (step S3). Accordingly, the notification unit 120 makes notification about the received information using a set method. For example, as illustrated in Fig. 8, the notification unit 120 causes the image monitor to display graphs of changes in electric power and impedance over time, together with a threshold value.

When the cauterization progresses over time, the biological impedance gradually increases. Since the electric power output by the energy generating unit 110 is maintained constant, when the impedance increases, the voltage output from the energy generating unit 110 automatically increases, and the current automatically decreases. Further, since the voltage output from the energy generating unit 110 is limited to a voltage value based a impedance threshold value set in advance, when the impedance is more than the threshold value, the voltage does not change and the current decreases, and as a result, the electric power decreases. For this reason, the atrial septum HA can be uniformly cauterized at a desired damage level. In addition, it is possible to reduce the generation of tissue damage caused by the application of excessive energy. In addition, it is possible to suppress the formation of blood clots caused by the application of excessive energy, and it is possible to reduce the generation of peripheral embolism or the like. In addition, when the atrial septum HA is cauterized to the desired damage level, the electric power output from the energy generating unit 110 automatically decreases to the extent that the atrial septum HA cannot be cauterized any more. For this reason, the notification unit 120 may be omitted.

The impedance calculation unit 132 of the control unit 130 determines whether or not the calculated impedance is more than the threshold value set in advance (step S4). Note that the impedance calculation unit 132 may determine whether or not the calculated impedance is equal to or more than the threshold value set in advance.
When it is determined that the impedance is more than the threshold value, the impedance calculation unit 132 causes the output (electric power) from the energy generating unit 110 to decrease (step S5). For example, the impedance calculation unit 132 causes the output from the energy generating unit 110 to immediately stop or to gradually decrease and stop. Accordingly, the maintenance treatment is completed. Note that instead of causing the output from the energy generating unit 110 to stop, the impedance calculation unit 132 may control the energy generating unit 110 to maintain the voltage constant, so that the output decreases more than when the output automatically decreases.

Thereafter, the information output unit 133 of the control unit 130 transmits information including that the impedance is more than the threshold value (or the threshold value or more) and that the impedance calculation unit 132 causes the output from the energy generating unit 110 to decrease, to the notification unit 120 (step S6). The notification unit 120 makes notification about the information using a set method.

Note that the control unit 130 can also cause the output from the energy generating unit 110 to stop, using a method different from the aforementioned control flow. For example, the operator may be able to forcibly stop or decrease the output from the energy generating unit 110 on his or her own judgement using input means such as a switch provided in the energy generating device 100 or in the cauterization device 10.

By the way, when the electrodes 22A to 22D are not in contact with a biological tissue, the impedance does not increase to the threshold value, so that the output (electric power) from the energy generating unit 110 does not decrease. For this reason, energy from the energy generating unit 110 continues to be applied, so that a risk of generation of blood clots increases. Therefore, when the impedance calculated by the impedance calculation unit 132 does not increases to the threshold value within a specified time set in advance, the control unit 130 causes the notification unit 120 to make notification about warning. Namely, the specified time is a time threshold value. The specified time is not particularly limited and is, for example, 20 to 40 seconds. Further, when the impedance does not increase to the threshold value within the specified time, the control unit 130 may forcibly cause the output from the energy generating unit 110 to stop.

After the maintenance treatment, the operator confirms hemodynamics again, and when the amount of the blood passing through the through-hole Hh reaches a desired amount, the operator reduces the expansion body 21 in diameter, stores the expansion body 21 in the storage sheath 30, and then removes the storage sheath 30 from the through-hole Hh. Further, the operator removes the entirety of the cauterization device 10 out of the living body to end the treatment.

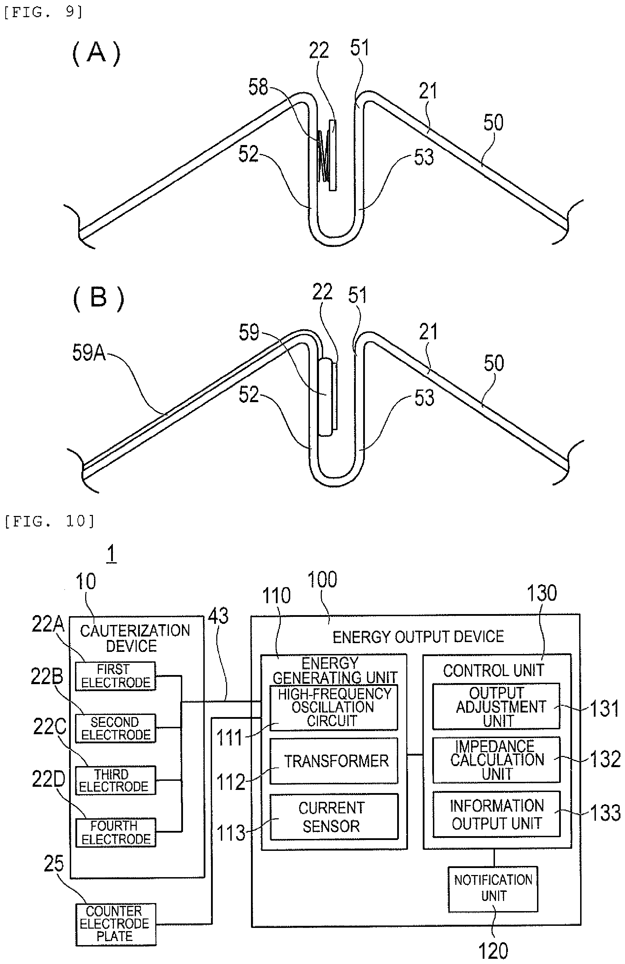

Note that as a first modification example, as illustrated in Fig. 9(A), the expansion body 21 that is a pressing mechanism may include spring elements 58 that bias the energy output unit 22 including the electrodes 22A to 22D toward the distal side holding portion 53, at the proximal side holding portions 52. The spring element 58 is, for example, a coil spring or a leaf spring. Accordingly, the energy output unit 22 supported by the spring elements 58 can be buried in the atrial septum HA. For this reason, the electrodes 22A to 22D of the energy output unit 22 are unlikely to come into contact with the blood during the maintenance treatment, so that it is possible to suppress the generation of blood clots or the like caused by the leakage of a current to the blood. Note that when the electrodes 22A to 22D are provided at the distal side holding portions 53, the spring elements 58 are provided at the distal side holding portions 53.

In addition, as a second modification example, as illustrated in Fig. 9(B), the expansion body 21 that is a pressing mechanism may include support balloons 59 that support the energy output unit 22 including the electrodes 22A to 22D, at the proximal side holding portions 52. A fluid is supplied to the support balloons 59 via a fluid supply tube 59A, so that the support balloons 59 can be inflated to move the energy output unit 22 toward the distal side holding portions 53. A proximal portion of the fluid supply tube 59A is led out from the operation unit 23 and is connectable to a syringe or the like that supplies the fluid. When the support balloons 59 is inflated, the electrodes 22A to 22D supported by the support balloons 59 can be buried in the atrial septum HA. For this reason, the electrodes 22A to 22D are unlikely to come into contact with the blood during the maintenance treatment, so that it is possible to suppress the generation of blood clots or the like caused by the leakage of a current to the blood. Note that when the electrodes 22A to 22D are provided at the distal side holding portions 53, the support balloons 59 are provided at the distal side holding portions 53.

In addition, as a third modification example, as illustrated in Fig. 10, the energy output unit 22 may be configured as a monopolar electrode. In this case, the energy generating unit 110 applies the same voltage to the first electrode 22A, to the second electrode 22B, to the third electrode 22C, and to the fourth electrode 22D. In addition, the energy generating unit 110 also applies a voltage to a return electrode plate 25 that is an electrode serving as a return electrode of the first electrode 22A, of the second electrode 22B, of the third electrode 22C, and of the fourth electrode 22D. Then, the first electrode 22A, the second electrode 22B, the third electrode 22C, and the fourth electrode 22D are energized with the return electrode plate 25 affixed to a body surface of a patient. When each of the electrodes 22A to 22D is configured as a monopolar electrode, the number of the electrodes 22A to 22D excluding the return electrode plate 25 is not particularly limited as long as the number of the electrodes 22A to 22D is 1 or more. In addition, the same voltage is applied to the first electrode 22A, to the second electrode 22B, to the third electrode 22C, and to the fourth electrode 22D, but different voltages may be applied thereto. Namely, the first electrode 22A, the second electrode 22B, the third electrode 22C, and the fourth electrode 22D may be separately controlled. In this case, the control unit 130 can separately control the first electrode 22A, the second electrode 22B, the third electrode 22C, and the fourth electrode 22D according to the aforementioned control flow (refer to Fig. 7).

As described above, the energy generating device 100 in the first embodiment includes the control unit 130 that controls an electric energy output to at least one of the electrodes 22A to 22D that cauterize a biological tissue. The control unit 130 includes the impedance calculation unit 132 that calculates an impedance between an electrode and an electrode serving as a return electrode of the electrode, and the output adjustment unit 131 that causes electric power to the electrodes to decrease when the impedance calculated by the impedance calculation unit 132 is more than a predetermined threshold value or is the threshold value or more. Accordingly, the energy generating device 100 can determine a damage level of the biological tissue based on an increase in the impedance of the cauterized biological tissue, and cause electric power to the electrodes 22A to 22D to automatically decrease. For this reason, the energy generating device 100 can uniformly cauterize the biological tissue at a desired damage level. In addition, it is possible to reduce the generation of tissue damage caused by the application of excessive energy, or the generation of peripheral embolism caused by the formation of blood clots.

In addition, when the impedance calculated by the impedance calculation unit 132 is the threshold value or less or is less than the threshold value, the output adjustment unit 131 causes electric power between an electrode and an electrode serving as a return electrode of the electrode, to be maintained substantially constant. Accordingly, the energy generating device 100 can reduce a decrease in tissue cauterization effect caused by a decrease in electric power, before the impedance reaches the threshold value, and facilitates uniform cauterization of the biological tissue at a desired damage level.

In addition, the cauterization system 1 according to the present embodiment includes the energy generating device 100, and the cauterization device 10 including at least one of the electrodes 22A to 22D that cauterize a biological tissue. Accordingly, the cauterization system 1 can uniformly cauterize the biological tissue at a desired damage level. In addition, it is possible to reduce the generation of tissue damage caused by the application of excessive energy, or the generation of peripheral embolism caused by the formation of blood clots.

In addition, the cauterization device 10 includes a pressing mechanism on which the electrodes 22A to 22D are disposed and by which the electrodes 22A to 22D are pressable against the biological tissue. Accordingly, the electrodes 22A to 22D come into close contact with the biological tissue, and are unlikely to be exposed in the blood. For this reason, it is possible to suppress the generation of blood clots or the like caused by the leakage of a current to the blood. In addition, since the electrodes 22A to 22D come into close contact with the biological tissue, a change in impedance can be accurately detected, so that a damage level of the tissue can be accurately determined. For this reason, the cauterization system 1 can uniformly cauterize the biological tissue at a desired damage level.

In addition, the cauterization device 10 includes the shaft portion 20 that is elongate. The pressing mechanism includes the expansion body 21 provided at the distal portion of the shaft portion 20 to be expandable and contractable in the radial direction. The expansion body 21 includes the plurality of wire portions 50 connected to the shaft portion 20, and at least one holding portion 51 formed of at least one wire portion 50. The holding portion 51 includes the distal side holding portion 53 and the proximal side holding portion 52 between which a separated distance is narrowed when the expansion body 21 expands. The electrodes 22A to 22D are disposed on the distal side holding portions 52 and/or on the proximal side holding portions 52. Accordingly, the cauterization system 1 effectively brings the electrodes 22A to 22D into close contact with the biological tissue using the expansion body 21, so that the electrodes 22A to 22D can be unlikely to be exposed in the blood.

In addition, the cauterization device 10 is a device that cauterizes a biological tissue in the vicinity of the through-hole Hh to maintain the through-hole Hh formed in the atrial septum HA. Accordingly, the cauterization system 1 can uniformly cauterize the biological tissue in the vicinity of the through-hole Hh of the atrial septum HA at a desired damage level to properly maintain the through-hole Hh.

### <Second Embodiment>

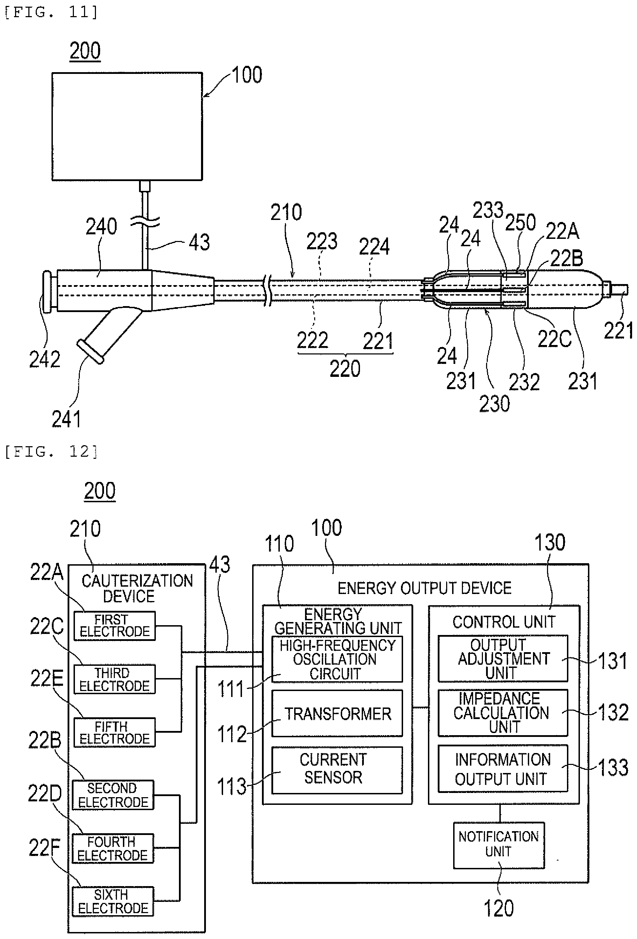

As illustrated in Figs. 11 and 12, a cauterization system 200 according to a second embodiment includes a cauterization device 210 different from that of the first embodiment. Note that portions having the same functions as those of the first embodiment are denoted by the same reference signs, and a description thereof will be omitted.

The cauterization device 210 includes a shaft portion 220 that is elongate, a balloon 230 provided at a distal portion of the shaft portion 220, and a catheter hub 240 connected to a proximal portion of the shaft portion 220. The balloon 230 is provided with an energy output unit 250 for performing cauterization.

The shaft portion 220 includes an outer tube 221 that is a tubular body of which a distal end and a proximal end are open, and an inner tube 222 disposed inside the outer tube 221. An inflation lumen 223 through which an inflation fluid for inflating the balloon 230 flows is formed between the outer tube 221 and the inner tube 222. A guide wire lumen 224 into which the guide wire 11 can be inserted is formed inside the inner tube 222.

A distal side of the balloon 230 is bonded to the inner tube 222 and a proximal side of the balloon 230 is bonded to the outer tube 221. The inside of the balloon 230 communicates with the inflation lumen 223. A distal side region and a proximal side region of the balloon 230 in the axial direction are high compliance portions 231 that are flexible and are easy to inflate. In addition, a region of an axially central portion of the balloon 230 is a low compliance portion 232 that is hard and difficult to inflate. An imaging marker 233 having an X-ray imaging property (X-ray opacity) is disposed on the low compliance portion 232.

The catheter hub 240 includes a first opening portion 241, a second opening portion 242, and the connector 43 that is connectable to the energy generating device 100. The first opening portion 241 communicates with the inflation lumen 223 of the outer tube 221 to allow the inflow and discharge of the inflation fluid. The second opening portion 242 communicates with the guide wire lumen 224.

The energy output unit 250 is disposed on an outer surface of the low compliance portion 232 of the balloon 230. The energy output unit 250 includes the first electrode 22A, the second electrode 22B, the third electrode 22C, the fourth electrode 22D, a fifth electrode 22E, and a sixth electrode 22F. The first electrode 22A, the second electrode 22B, the third electrode 22C, the fourth electrode 22D, the fifth electrode 22E, and the sixth electrode 22F are disposed side by side at substantially equal intervals in order on an outer peripheral surface of the low compliance portion 232.

The energy output unit 250 is configured as a bipolar electrode that receives electric energy from the energy generating device 100.
The same voltage is applied to the first electrode 22A, to the third electrode 22C, and to the fifth electrode 22E. The same voltage is applied to the second electrode 22B, to the fourth electrode 22D, and to the sixth electrode 22F. A voltage having a polarity opposite that of the first electrode 22A, of the third electrode 22C, and of the fifth electrode 22E is applied to the second electrode 22B, to the fourth electrode 22D, and to the sixth electrode 22F. Therefore, energization is performed between the first electrode 22A and the second electrode 22B, between the second electrode 22B and the third electrode 22C, between the third electrode 22C and the fourth electrode 22D, between the fourth electrode 22D and the fifth electrode 22E, between the fifth electrode 22E and the sixth electrode 22F, and between the sixth electrode 22F and the first electrode 22A. Electric energy from the connector 43 connected to the energy generating device 100 is supplied to the energy output unit 250 by the conducting wire 24 coated with an insulating coating material.
Note that the energy output unit 250 may be configured as a monopolar electrode to be used together with a return electrode plate.

It is preferable that the balloon 230 is made of a material having a certain degree of flexibility, and examples of such a material include polyolefins such as polyethylene, polypropylene, polybutene, ethylene-propylene copolymer, ethylene-vinyl acetate copolymer, ionomer, and a mixture of two or more thereof, thermoplastic resins such as soft polyvinyl chloride resin, polyamide, polyamide elastomer, polyester, polyester elastomer, polyurethane, fluororesin, silicone rubber, and latex rubber.

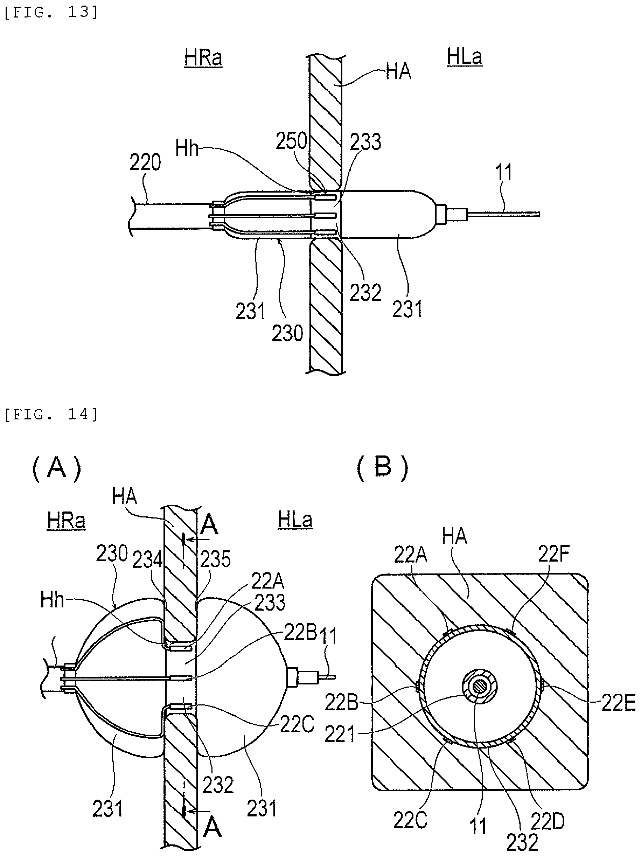

As illustrated in Fig. 13, an operator can accurately dispose the low compliance portion 232 in the through-hole Hh of the atrial septum HA while confirming the position of the imaging marker 233 under radioscopy. Thereafter, when the balloon 230 is inflated, as illustrated in Fig. 14, surfaces on center sides of the high compliance portions 231 face each other while interposing the low compliance portion 232 therebetween. The surfaces facing each other serve as a proximal side holding portion 234 and a distal side holding portion 235 that interpose the atrial septum HA therebetween. Since the proximal side holding portion 234 and the distal side holding portion 235 interpose the atrial septum HA therebetween, the low compliance portion 232 can be inflated without being displaced from the through-hole Hh, to widen the through-hole Hh. Then, when the low compliance portion 232 is inflated, the electrodes 22A to 22F disposed on the low compliance portion 232 are strongly pressed against the through-hole Hh. Accordingly, in a state where the electrodes 22A to 22F are buried in a biological tissue, the maintenance treatment is performed. Accordingly, the electrodes 22A to 22F do not come into contact with the blood during the maintenance treatment, so that it is possible to suppress the generation of blood clots or the like caused by the leakage of a current to the blood.

As described above, the cauterization device 210 of the cauterization system 200 according to the second embodiment includes the shaft portion 220 that is elongate. The pressing mechanism includes the balloon 230 provided at the distal portion of the shaft portion 220 to be inflatable and contractable in the radial direction. The electrodes 22A to 22F are disposed on the outer surface of the balloon 230. Accordingly, the energy generating device 100 effectively brings the electrodes 22A to 22F into close contact with the biological tissue using the balloon 230, so that the electrodes 22A to 22D can be unlikely to be exposed in the blood.

Note that the invention is not limited only to the aforementioned embodiments and various modifications can be made by those skilled in the art without departing from the technical concept of the invention.
For example, the cauterization system is not limited to a system that maintains the through-hole Hh formed in the atrial septum HA. For example, the cauterization system may be a system that cauterizes a myocardium to treat arrhythmia, a system that cauterizes and cuts sympathetic nerves to treat hypertension, or the like. In addition, the output adjustment unit 131 of the control unit 130 may cause the energy generating unit 110 to output a high-frequency current at a constant voltage. In this case, since the voltage output by the energy generating unit 110 is maintained constant, when the cauterization progresses over time and the impedance increases, the electric power output from the energy generating unit 110 automatically decreases. For this reason, it is possible to decrease energy applied to the atrial septum HA in a high impedance region, and it is possible to suppress the carbonization of tissues caused by rapid heat generation and the generation of bubbles caused by water vapor. Also in this case, when the impedance is more than a predetermined threshold value or is the threshold value or more, the output adjustment unit 131 forcibly causes electric power output by the energy generating unit 110, to decrease. Accordingly, a biological tissue can be uniformly cauterized at a desired damage level. In addition, it is possible to reduce the generation of tissue damage caused by the application of excessive energy, or the generation of peripheral embolism caused by the formation of blood clots.

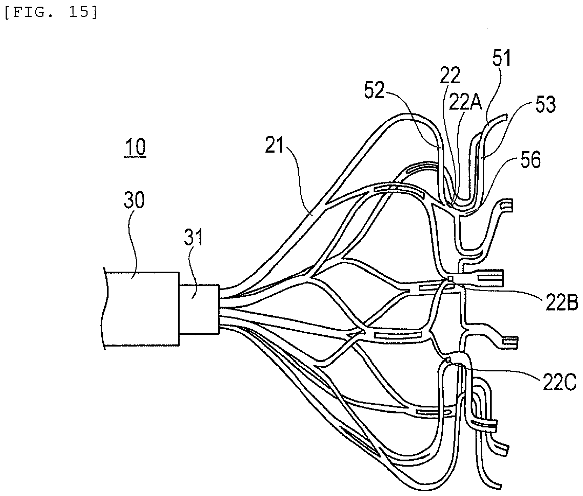

In addition, as a modification example illustrated in Fig. 15, the expansion body 21 may be formed in a mesh shape where wire portions are branched and merged together. The expansion body 21 includes a plurality of the holding portions 51 that are recessed portions, and the energy output unit 22 that is an electrode is disposed on the proximal side holding portion 52 of each of the holding portions 51. Note that the position where the energy output unit 22 is disposed is not limited to the proximal side holding portion 52 and may be a position on the distal side holding portion 53 or on the inward projection portion 56, at which the energy output unit 22 can come into contact with a biological tissue. In the present modification example, the pulling shaft is not provided. Therefore, the expansion body 21 released from the storage sheath 30 expands a puncture hole Hh using only its own expansion force.

Note that this application is based on Japanese Patent Application No. 2019-179469, filed on September 30, 2019, the content of which is incorporated herein by reference in its entirety.

### Reference Signs List

1, 200: Present cauterization system
10, 210: Cauterization device
20, 220: Shaft portion
21: Expansion body (pressing mechanism)
22, 250: Energy output unit
22A: First electrode
22B: Second electrode
22C: Third electrode
22D: Fourth electrode
22E: Fifth electrode
22F: Sixth electrode
25: Return electrode plate
50: Wire portion
51: Holding portion
52, 234: Proximal side holding portion
53, 235: Distal side holding portion
58: Spring element
59: Support balloon
100: Energy generating device
110: Energy generating unit
120: Notification unit
130: Control unit
131: Output adjustment unit
132: Impedance calculation unit
133: Information output unit
230: Balloon (pressing mechanism)
HA: Atrial septum
Hh: Through-hole

## Claims

1. An energy generating device comprising:
a control unit that is configured to control an electric energy output to at least one electrode configured to cauterize a biological tissue,
wherein the control unit includes an impedance calculation unit that is configured to calculate an impedance between the electrode and an electrode serving as a return electrode of the electrode, and
an output adjustment unit that is configured to cause electric power to the electrodes to decrease when the impedance calculated by the impedance calculation unit is greater a predetermined threshold value or is equal to or greater the threshold value.

2. The energy generating device according to claim 1,
wherein the output adjustment unit is configured to cause the electric power between the electrode and the electrode serving as a return electrode of the electrode, to be maintained substantially constant when the impedance calculated by the impedance calculation unit is equal to or less than the threshold value or is less than the threshold value.

3. The energy generating device according to claim 1,
wherein the output adjustment unit is configured to cause a voltage between the electrode and the electrode serving as a return electrode of the electrode, to be maintained substantially constant when the impedance calculated by the impedance calculation unit is equal to or less than the threshold value is less than the threshold value.

4. A cauterization system comprising:
the energy generating device according to any one of claims 1 to 3; and
a cauterization device including at least one electrode that is configured to cauterize a biological tissue.

5. The cauterization system according to claim 4,
wherein the cauterization device includes a pressing mechanism on which the electrode is disposed and by which the electrode is pressable against the biological tissue.

6. The cauterization system according to claim 5,
wherein the cauterization device includes an elongated shaft portion,
the pressing mechanism includes an expansion body provided at a distal portion of the shaft portion to be expandable and contractable in a radial direction,
the expansion body includes a plurality of wire portions connected to the shaft portion, and at least one holding portion formed of at least one wire portion,
the holding portion includes a distal side holding portion and a proximal side holding portion between which a separated distance is narrowed when the expansion body expands, and
the electrode is disposed on the distal side holding portion and/or on the proximal side holding portion.

7. The cauterization system according to claim 5,
wherein the cauterization device includes an elongated shaft portion,
the pressing mechanism includes a balloon provided at a distal portion of the shaft portion to be inflatable and contractable in a radial direction, and
the electrode is disposed on an outer surface of the balloon.

8. The cauterization system according to any one of claims 4 to 7,
wherein the cauterization device is a device that configured to cauterize a biological tissue in the vicinity of a through-hole to maintain the through-hole formed in an atrial septum.
